# EUROPEAN PATENT APPLICATION

(11) **EP 4 793 970 A2**
(43) Date of publication of application: **19.08.2026**
(21) Application number: 26190379.3
(22) Date of filing: 22.02.2023
(51) Int. Cl.: G16H 40/67

(54) **MANAGING CONTAINERS IN A MEDICAL DIAGNOSTIC SYSTEM**

(30) Priority: 23.02.2022 US 202217678831; 08.06.2022 LU 102964
(62) Document number(s) of the earlier application(s) in accordance with Art. 76 EPC: 23157991.3 / 4 233 774
(71) Applicant: Instrumentation Laboratory Company, Bedford, MA 01730 (US)
(72) Inventor: FARREN, Christopher, Bedford, 01730 (US); ATKINS, Adam, Bedford, 01730 (US); SHREVE, Joshua, Bedford, 01730 (US); NEWTON, Benjamin, Bedford, 01730 (US); BASSIK, Renen, Bedford, 01730 (US); CIEMNY, Jonas, Bedford, 01730 (US); AKAGLA, Linda Elizabeth, Bedford, 01730 (US)
(74) Representative: Müller-Boré & Partner Patentanwälte PartG mbB

(57) **Abstract**

An example medical diagnostic system includes one or more containers that are usable in a medical diagnostic test. Each container has an identification (ID) tag associated therewith. An ID tag of a container includes memory that is readable and writeable. The memory is for storing information relating to the container. Antennas are configured to communicate wirelessly with ID tags associated with the one or more containers. A control system is configured to store a location of the container based on an identify of the antenna, to select the antenna for communication with the ID tag, and to use the antenna to read at least some of the information from, or to write at least some of the information to, the memory on the ID tag.

## Description

### TECHNICAL FIELD

This specification relates generally to example medical diagnostic systems that use electronic identification tags to manage containers storing substances.

### BACKGROUND

A medical diagnostic system performs tests on a sample, such as blood or tissue, obtained from a patient. The tests performed by the medical diagnostic system are referred to as assays. An example assay is an investigative procedure for qualitatively assessing or quantitatively measuring the presence, amount, or functional activity of an analyte in the sample. One or more substances, such as reagents, controls, or calibrators, may be used by the medical diagnostic system to perform an assay on a sample.

### SUMMARY

An example medical diagnostic system includes one or more containers that are usable in a medical diagnostic test. Each container has an identification (ID) tag associated therewith. An ID tag of a container includes memory that is readable and writeable. The memory is for storing information relating to the container. Antennas are configured to communicate wirelessly with ID tags associated with the one or more containers. A control system is configured to store a location of the container based on an identify or identity of the antenna, to select the antenna for communication with the ID tag, and to use the antenna to read at least some of the information from, or to write at least some of the information to, the memory on the ID tag. The medical diagnostic system may include one or more of the following features, either alone or in combination.

An antenna among the antennas may be within communication range of the ID tag of the container. The control system may include a multiplexer that is controllable to select the antenna and a read/write module to implement reading at least some of the information from, or writing at least some of the information to, the memory on the ID tag. One or more of the containers may be arranged in an array. The antennas may be arranged in a static array that is along the array of containers such that each container is in physical correspondence with an antenna in the static array and such that an ID tag of each container is in communication range of an antenna in the static array.

The information relating to the container may include one or more of: a state of the container, calibration data for the container, identification data for the container, container manufacturer location, container expiration date, material consumed in the container, onboard stability information, assay data for the medical diagnostic test, container authenticity data, or error detection data.

The medical diagnostic system may be a first medical diagnostic system. The information in the memory on the ID tag may be usable by a second medical diagnostic system that is separate from the first medical diagnostic system to incorporate the container into a medical diagnostic test performed by the second medical diagnostic system. The medical diagnostic system may include a mobile robot that is configured to move relative to the one or more containers. An antenna may be mounted to the mobile robot to cause the antenna to move relative to the one or more containers and, based on the movement, to communicate wirelessly with the ID tag. The control system may be configured to control movement of the mobile robot.

An example mobile robot includes a gripper for grasping a container in a medical diagnostic system and a mount for holding an antenna configured to communicate wirelessly with electronic identification (EID) tags on containers in the medical diagnostic system. The mobile robot is configured to move the container between different areas of the medical diagnostic system. The mobile robot may include one or more of the following features, either alone or in combination.

The mobile robot may include an element to apply force to a part of the container during movement in order to stabilize the container during the movement. The element may include a spring-loaded plate. The mobile robot may be configured to move in three dimensions. The mobile robot may include a switch that detects engagement with the container. The switch may be configured to output a signal in response to engagement between the container and the mobile robot.

An example system includes the mobile robot and a control system to control operation of the mobile robot. The control system may be configured to detect the signal output by switch. The control system may be configured to track movement of the mobile robot relative to the container, to detect that the signal was not output when expected, and to output an error signal or message in response to detecting that the signal was not output when expected. The system may include a robotic laboratory tool configured to move between containers to transfer a substance from a first container to a second container. The control system may be configured to control movement of the mobile robot so that that mobile robot is out of the way of the robotic laboratory tool during operation of the robotic laboratory tool.

An example user interface (UI) for a medical diagnostic system includes a menu for selecting one or more containers in the medical diagnostic system based on a substance in the containers or a status of the containers, and a display containing information about the one or more containers selected. The UI may include one or more of the following features, either alone or in combination.

The one or more containers may be represented graphically on the UI. The information may include at least one of the following: locations of the one or more containers in an area of the medical diagnostic system, container identifiers, container lot identifiers, an indication that a container has loaded into the medical diagnostic system, an indication that a container is active in the medical diagnostic system, a number of tests remaining in a container in the medical diagnostic system, locations of empty slots not containing a container in the medical diagnostic system, container capacity of the medical diagnostic system, or a number of each sized container in the medical diagnostic system.

An example method for use in a medical diagnostic system includes the following operations: selecting a first antenna among multiple antennas, where the first antenna is associated with an identification (ID) tag of a container that is usable in a medical diagnostic test, where the ID tag includes memory that is readable and writeable, and where the memory is for storing information relating to the container; and performing at least one of the following operations: reading at least some of the information from the memory using the first antenna, or writing at least some of the information to the memory using the first antenna. The operations in the example method may also include moving the container from a first location of the medical diagnostic system to a second location of the medical diagnostic system using a mobile robot; and communicating with the ID tag of the container using a second antenna mounted to the mobile robot at least to update one or more of: the memory on the ID tag or a control system to provide information from the ID tag.

The method may include one or more of the following features, either alone or in combination: storing, in memory, a first location of the container based on an identity of the first antenna; and storing, in memory, the second location of the container.

Any two or more of the features described in this specification, including in this summary section, can be combined to form implementations not specifically described herein.

The systems, techniques, components, structures, and variations thereof described herein, or portions thereof, can be implemented using, or controlled by, a computer program product that includes instructions that are stored on one or more non-transitory machine-readable storage media, and that are executable on one or more processing devices to execute at least some of the operations described herein. The systems, techniques, components, structures, and variations thereof described herein, or portions thereof, can be implemented as an apparatus, method, or electronic system that can include one or more processing devices and computer memory to store executable instructions to implement various operations. The systems, techniques, components, structures, and variations thereof described herein may be configured, for example, through design, construction, size, shape, arrangement, placement, programming, operation, activation, deactivation, and/or control.

The details of one or more implementations are set forth in the accompanying drawings and the description below. Other features, objects, and advantages will be apparent from the description and drawings, and from the claims.

### DESCRIPTION OF THE DRAWINGS

Fig. 1 is block diagram showing components of an example medical diagnostic system that uses electronic identification (EID) tags to manage containers.
Fig. 2 is a perspective view of an example container and structure of an EID tag on, or associated with, the container.
Fig. 3 is a perspective view of an example area of the medical diagnostic system that includes arrays of radio frequency (RF) antennas and containers along with circuitry for communicating with the RF antennas.
Fig. 4 is a perspective view of example area containing arrays of containers and an example mobile robot configured to sweep over an array to communicate with electronic identification tags on or associated with containers in the array.
Fig. 5, comprised of Figs. 5A and 5B, is a flowchart showing example operations that may be performed by or on the medical diagnostic system.
Fig. 6 is a flowchart showing example operations that may be performed by or on the medical diagnostic system.
Fig. 7, comprised of Figs. 7A and 7B, shows example windows of an example user interface (UI) that may be generated by the medical diagnostic system.
Fig. 8 is a front view of components of an example mobile robot that may be usable in the medical diagnostic system.
Fig. 9 is a back perspective view of part of the example mobile robot of Fig. 8.
Like reference numerals in different figures indicate like elements.

### DETAILED DESCRIPTION

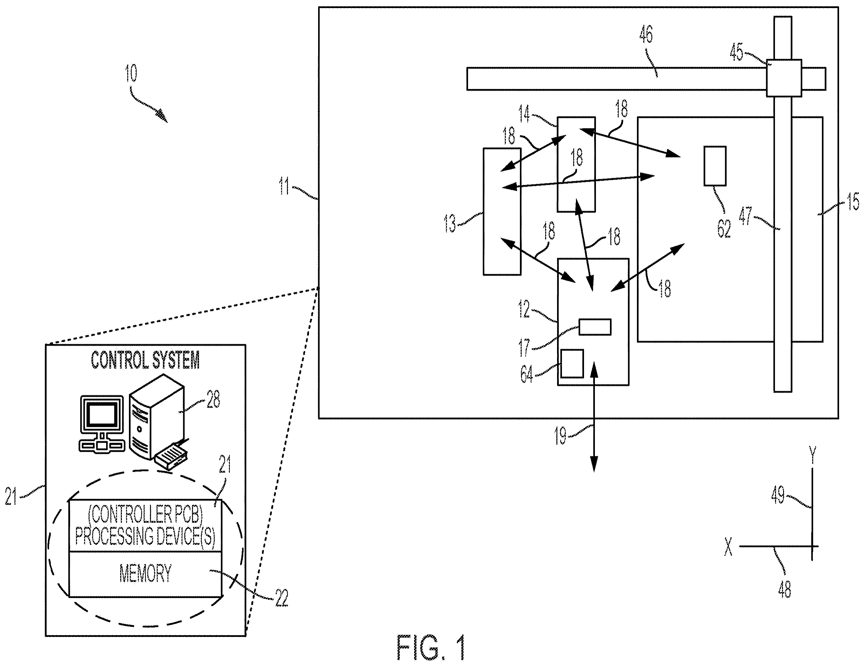

Described herein are examples of medical diagnostic instruments, systems, and variants thereof (referred to collectively as "the system") that use electronic identification (ID) tags to manage containers storing substances that are usable to perform diagnostic tests or assays. Fig. 1 shows a block diagram of components of an example system 10 of this type. Example system 10 includes a medical diagnostic instrument 11 containing four areas 12, 13, 14, and 15. Containers, such as container 17, may be moved among the four areas as described herein. The arrows, collectively referred to as 18, show examples of potential directions of movement of container 17 within instrument 11. Arrow 19 shows directions of movement of container 17 into or out of instrument 11.

In a non-limiting example, area 12 may include an input/output (I/O) drawer and areas 13, 14, and 15 may be temperature-controlled. In some examples, the I/O drawer is a user-accessible drawer into which containers may be loaded. In some examples, the temperature-controlled areas include ambient temperature areas and/or chilled areas. In some examples, there may be more than four areas - for example, five, six, or seven areas - or fewer than four areas - for example, two or three areas.

In some implementations, the containers may be or include a test cartridge ("cartridge") containing multiple vials, one or more of which is configured to hold a substance that may be used in testing. However other types of containers may be used such as, but not limited to, cuvettes or stand-alone vials. Examples of substances in the containers may include, but are not limited to, individual, or mixtures of, reagents in dry or liquid form, control substances, diluents, DI (deionized) water, cleaning materials, and/or calibrators.

In some implementations, each container has an electronic identification (EID) tag attached thereto or associated therewith. Examples of EID tags include, but are not limited to, a radio frequency (RF) ID tag ("RFID tag") and an electrically erasable programmable read-only memory (EEPROM). Examples of types of RFID tags that may be used include, but are not limited to, low frequency RFID tags (e.g., an RFID tag having a transmission rate of about 125 kilohertz to 134 kilohertz (KHz)), high frequency RFID tags (e.g., an RFID tag having a transmission rate of about 13.56 megahertz (MHz)), and ultra-high frequency RFID tags (e.g., an RFID tag having a transmission rate of about 860 MHZ to 960 MHz). These are examples only; the system is not limited to use with these types of EID or RFID tags.

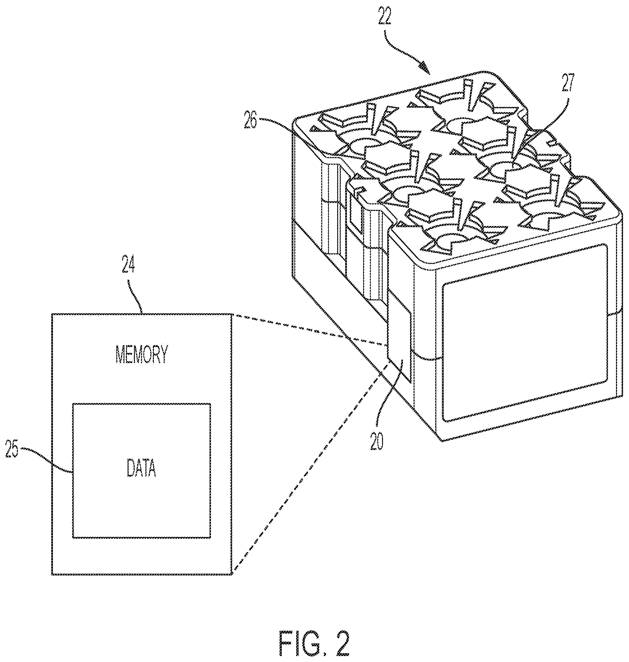

The EID tag contains memory that is machine-readable and machine-writeable (referred to as "memory"). The memory may store information relating to the container for managing the container and/or tests associated with the container, such as the assay that the container is used for. Referring to Fig. 2 an example of an EID tag 20 is mounted to, or otherwise connected to, a side of container 22. EID tag 20 includes memory 24 to store data 25 (described below). In some implementations, the memory may be on the order of hundreds of bytes of data, such as 100 bytes, 200 bytes, 300 bytes, 400 bytes, 500 bytes, or more. Any amount of memory may be included on an EID tag used in the system. Data may be read from, and/or written to, the memory wirelessly using RFID antennas, as described below. The example container 22 of Fig. 2 includes an outer shell 26 holding multiple vials such as 27.

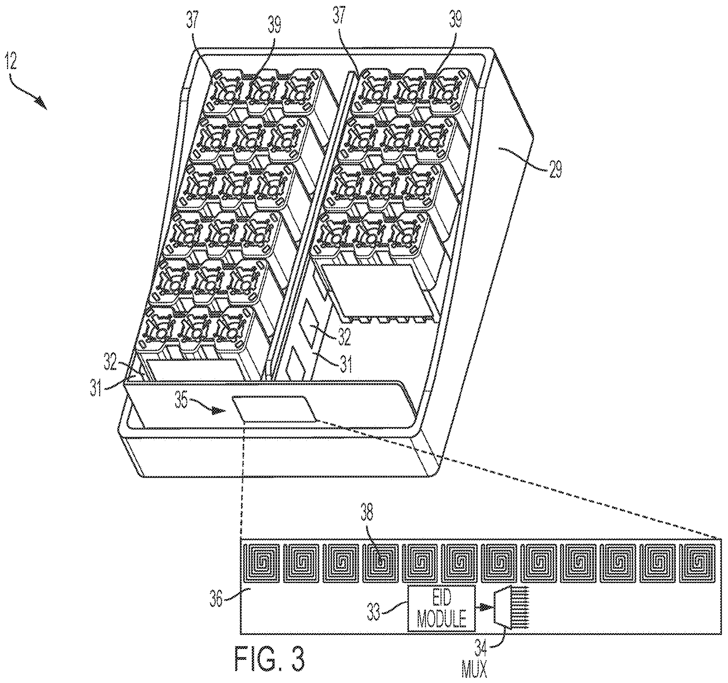

EID tags may be located on one side of each container as shown in Figs. 2 and 3 or on both sides of each container (not shown). By including an EID tag on both sides of each container, along with corresponding RFID antennas to read those EID tags, the system may enable reading and writing data to/from the EID tags regardless of the orientation at which the container is placed within an area. The additional EID tags may also provide additional storage. For example, the additional storage provided by additional EID tags may store additional information and may be beneficial if memory size is limited by the physical dimensions of the EID tags that fits on a container. There may also be a lower costs associated with lower-storage EID tags.

Referring to Fig. 3, area 12 includes I/O drawer 29 in this example, but any of the areas 12 to 15 may have the antenna configuration shown in Fig. 3. In area 12, there are arrays of RF antennas 32. Both arrays of antennas are only partially visible in this example and are configured - for example, arranged - to align to and to face corresponding EID tags on the sides 37 of the two arrays 39 of containers. The antennas are referred to as "static" since, in this example, they do not move relative to the containers in area 12. In this example, the RF antennas are configured to use HF (high frequency) NFC technology to communicate with the EID tags. NFC is a set of communication protocols that enables wireless communication between electronic devices over a short distance. In this example, the distance between an RF antenna and corresponding EID tag is between one and two centimeters (cm) - e.g., about 12 millimeters (mm) - however, the system is not limited to communication over this range of distances. For example, in some implementations, the enabled communication distance between an RF antenna and corresponding EID tag is 8mm, 10mm or less, or 15mm, 20mm, 30mm, 40mm, 50mm, or more. In any case, the antennas and the EID tags are arranged to be within the communication distance. In some implementations, the containers may have modularized sizes and, as a result, the RF antennas are repeated at the module distance, as shown with respect to antennas 32.

In this example, each RF antenna 32 is mounted to a printed circuit board (PCB) or flexible circuit 31. Each PCB or flexible circuit 31 is connected electrically to another PCB or flexible circuit 35 that contains EID circuitry 36. EID circuitry 36 may be considered part of a control system (described below) and may operate as the intermediate entity between the RF antennas and the remainder of the control system. In this example, EID circuitry 36 includes a multiplexer 34, an EID read/write module 33 ("EID module"), and electrical connections 38 to antennas 32, which connections may be considered part of the antennas. Multiplexer 34 creates a wired electrical connection between EID module 33 and antennas 32/38 via conductive traces (not shown) in PCBs 35, 31 for the purpose of enabling the EID module 33 to communicate wirelessly with a specific EID tag 32 (Fig. 3). In this example, EID module 33 includes a controller, such as programmable logic or a microcontroller, that is configured to control operation of the multiplexer.

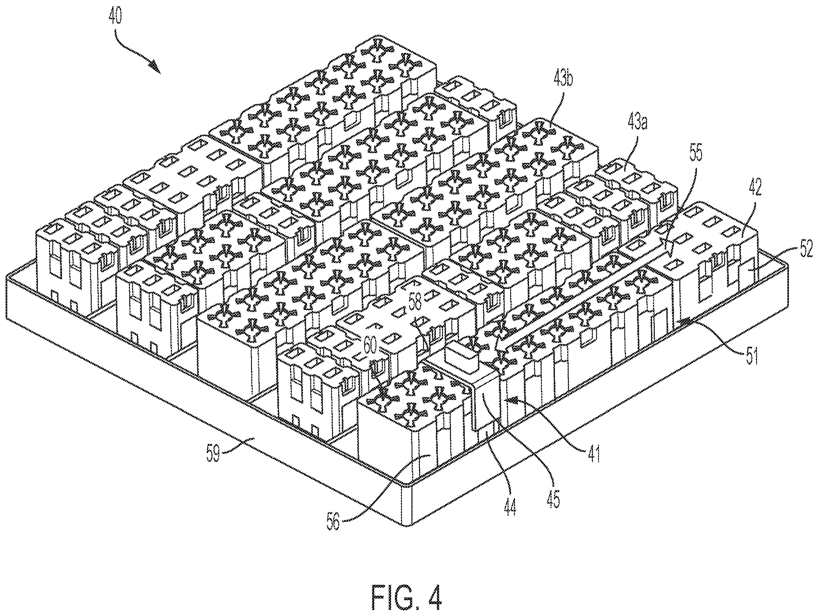

Referring to Fig. 4, in area 40, which may represent one or more of areas 13 to 15 of Fig. 1, there need not be static RF antennas. Instead, a mobile RF antenna 41 is configured to move relative to EID tags of containers 42 (e.g., EID tag 44) to read and/or to write to the memory on those EID tags. The mobile RF antenna can be used to implement the same reading and writing as the static antennas. The mobile RF antenna is mounted on or associated with a mobile robot 45 ("robot") used to pick-up a container in one area, to move the container to another area, and to place the container in the other area. In some implementations, the mobile RF antenna can also be mounted on a different mobile robot than the mobile robot used for picking-and-placing operations. The mounting makes the mobile RF antenna mobile. The example of Fig. 1 shows mobile robot 45 mounted to tracks 46 and 47 that enable it to access each area 12 to 15. For example, mobile robot 45 and track 47 may be physically connected and move together along track 46 in the X-dimension 48 and mobile robot 45 may move also along track 47 in the Y-dimension 49 following X-dimension positioning.

In the example of Fig. 4, robot 45, and thus mobile RF antenna 41, is configured to move relative to an array of containers 42. For example, the robot, and thus the mobile RF antenna, is configured to sweep over the array in the directions of arrow 55. The mobile robot may then be controlled to move over other arrays of containers such as arrays 43a and 43b. While robot 45 moves, its RF antenna 41 moves sequentially into communication range of the EID tags 44, 51, and 52 of each of containers 42 in the array. As RF antenna 41 comes within communication range of each EID tag, the RF antenna may read and/or write data such as that described herein to and/or from the EID tag. Movement of mobile RF antenna 41 is calibrated to enable NFC communication when its RF antenna is in range of the EID tags. That is, movement that is too quick may prevent, inhibit, or corrupt communication to and/or from the EID tags. In some implementations, the rate of movement of the robot (and thus of the RF antenna) may be constant and calibrated to enable NFC communications with the EID tags. In some implementations the robot may be controlled to move more quickly between EID tag locations and to move more slowly when in NFC communication range with an EID tag. In some implementations, the robot may be controlled to move between tag locations quickly and/or at a constant speed and to slow in speed or to stop at each EID tag location to implement the NFC communications with the EID tag. For example, the robot may be controlled to slow in speed or to stop to implement the reading or writing.

In some implementations, there may be two RF antennas on a single mobile robot 45. For example, there may be a first RF antenna 41 on one side of the robot and a second RF antenna (not shown in the figures) on the other side 58 of robot 45. Use of two RF antennas may reduce the need to maintain consistent orientation of the containers in a rack 59. For example, if container 60 is rotated by 180°, its EID tag 44 will be facing in the direction opposite to the direction that EID tag 44 faces in Fig. 4. By including an RF antenna on both sides of robot 45, the system may enable reading and writing data for different container orientations. For containers containing multiple EID tags, the advantages are the same as those described in the static implementation above.

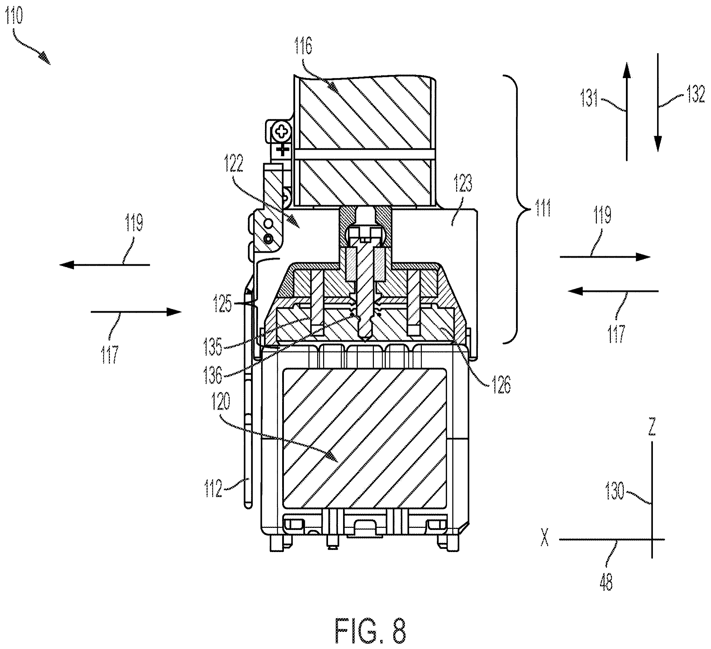

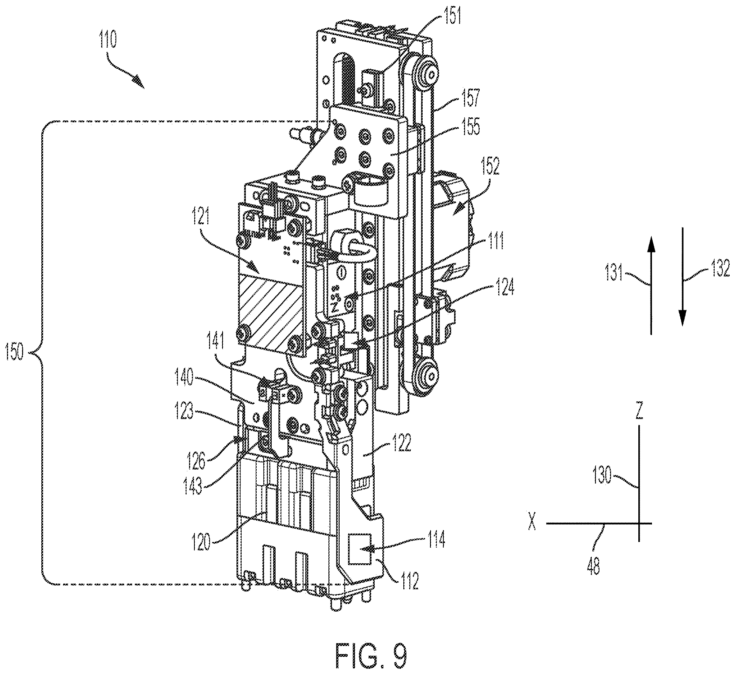

Figs. 8 and 9 show views of components of an example robot 110, which is an example implementation of robot 45 described above. Robot 110 includes a gripper 111 and a mount 112 for holding an RF antenna 114 (like RF antenna 41). An electrical motor 116, which is responsive to commands from the control system, controls fingers on the gripper to move in the directions of arrows 117 to engage a container 120 and in the directions of arrows 119 to release container 120. More specifically, the left 122 and right 123 fingers of gripper 111 may be controlled to move in the directions of arrows 117 to contact, and to apply gripping force to, container 120 and in the directions of arrows 119 to release that gripping force. Local control electronics, such as circuit board 121, may be configured to receive the control system commands and to implement gripper control. A switch 124 may be triggered when the fingers of the gripper are fully contracted, which may signal to the control system that the gripper has engaged the container and that the container is ready for transport.

In some implementations, the container includes one or more notches (not shown) on one or both sides thereof, and the left and/or right fingers of gripper 111 include one or more juts (not shown) having shapes that complement the shapes of the notches on the container. When the gripper engages the container, the juts enter the notches, ensuring proper alignment between the gripper and the container and providing additional structural support to enable the robot to lift the container.

Robot 110 also includes a plate assembly 125. Plate assembly 125 includes a plate 126 that is movable relative to container 120 and gripper 111 along Z-axis 130 in the directions of arrows 131, 132 and over pins or rods 135. In some implementations, the plate is spring 136 loaded and moves at the same time as the grippers. By way of example, when the gripper engages container 120, plate 126 contacts the top of container 120, causing spring 136 to compress. The left 122 and right 123 fingers of gripper 111 engage container 120 as described above, with plate 126 forced against the top of container 120 by force applied along the Z-axis in the the direction of arrow 132 by the compressed spring. This force applied by plate 126 during transport of the container reduces the chances that the container will move relative to the robot, thereby reducing the chances that the container contents will slosh or spill during transport.

As shown in Fig. 9, robot 110 also includes a height detection switch 140. Height detection switch may be or include an optical switch. The optical switch includes an optical element 141 that produces a light beam that, when blocked, sends an electrical signal to the control system. The electrical signal represents an indication that contact with the container has been made. The optical switch also includes a trigger 143. Trigger 143 may be an opaque plate or finger that blocks transmission of the light beam and, therefore, when placed in the light beam's path, blocks the light beam.

Trigger 143 may be physically attached to plate 126 and, therefore, moves in the Z dimension along with movement of plate 126. Trigger 143 and optical element 141 are positioned relative to each other and relative to plate 126 so that, when plate 126 compresses the spring by at least a predetermined amount, such as full compression, the trigger moves into the light beam, thereby blocking the light beam and causing output of the electrical signal. The electrical signal thus may act as confirmation to the control system that the robot has engaged a container. For example, the electrical signal indicates that the plate is at the appropriate height to pick-up the container.

In some implementations, if trigger 143 never triggers optical element 141 in an expected Z dimension range, then the system assumes there is no container present to grasp and pick. The system may attempt this motion one or more additional times to "search" for the container - for example, to see if trigger 143 triggers the optical element 141 in the expected Z dimension range - but the system will eventually shift to an error state or move on to a next task if there is no container present in the expected Z dimension. In some implementations, if trigger 143 triggers the optical element 141 before the expected Z dimension range, the system assumes the container is not seated correctly. Again, the system may attempt this motion one or more additional times before shifting to an error state or moving on to a next task.

In addition to the X and Y axis movement described with respect to Fig. 1, as noted part of robot 110 is also configured for Z-axis movement. To this end, robot 111 includes an assembly 150, a track 151, and a motor / encoder 152. Assembly 150 is connected to track 151 by coupling 155, which enables assembly 150 to move up (arrow 131) and down (arrow 132) track 151 along the Z-axis. Motor / encoder 152, which is responsive to commands from the control system, controls movement of assembly 150 along the track, records the location of assembly 150 along track 151, and provides that information to the control system. The motor / encoder 152 may control operation of a belt 157 connected to coupling 155 to control movement of assembly 150 along track 151. The Z-axis motion of part of robot 110 allows for the robot to retreat to a compact retracted position to provide both clearance over laboratory tools (described below) in an area and clearance between a container in transport and other system hardware and containers. The Z-axis motion also enables access to containers located inside one or more of the areas that are located at different depths relative to the robot.

Referring back to Fig. 1, one or more (e.g., all) of areas 13 to 15 may contain one or more robotic laboratory tools 62 (shown only in area 15 of Fig. 1), such as, but not limited to, a pipette or a probe, that are configured to operation in that area. An example laboratory tool may be configured to move automatically within its respective area to reach a container, to pierce or enter to container, to remove substance therefrom, and to transfer that substance to another container.

One or more (e.g., all) of areas 12 to 15 may contain a presence detector such as detector 64 (shown only in area 12). Presence detector 64 may be or include an optical sensor that detects the presence of at least one container in the area and that reports data to the control system indicating the container's presence.

System 10 includes control system 21 to control the automated components thereof. As shown in Fig. 1, in some implementations, control system 21 includes a controller PCB 21 having one or more processing devices of the type described herein that are programmable to control operations of automated components of system 10. Control system 21 may include memory 22 for storing data and programs executable by the controller PCB. The control system may also include a computing system 28 that communicates with the controller PCB or that is part of the controller PCB. Computing system 28 may be separate from instrument 11, but may be connected to instrument 11 directly or via a wired or wireless computer network to enable communication between the instrument and the computing system.

In some implementations, at least part of the control system, such as the controller PCB, may reside on instrument 11. For example, all or part of the control system may be embedded in or otherwise part of instrument 11. In some implementations, all or part of control system 21 may be distributed across instrument 11 and one or more computing devices that may be in communication with instrument 11. The control system is programmed to direct where and when to move containers from one location to another based, at least in part, on test/assay workflows stored either in the control system of the EID tag of a container..

The control system stores, in its software, a set of parameters, recipes, preparation steps, and position of containers with instrument 11 - basically any data the system uses to implement system workflow for the tests or assays to be implemented. Container descriptions associated with container identification codes, as described below, may also be stored in the control system. Each container description may be associated with its identification code. Accordingly, a container description may be retrieved using the identification code obtained from the EID tag. The control system thus knows what types information is stored on the EID tag based its identification code. The control system also stores the location of each container determined as described below and expected heights of the container at different locations within the instrument. This information is usable as described below to detect proper container placement. The manufactured fill volume of each container also may be stored on the control system and accessed using an identification code of the container. The control system may also store information identifying what substance(s) are is in each container and which container is in which position inside a cartridge.

The EID tag of each container, there is a single identification number or code that can be read from the EID tag by the control system, which will tell the control system what processes to implement for the container (e.g., recipes, sequences, various lookup tables, and the like). Basically any piece of information that is relatively constant over time (not lot specific) and that is used to inform the control system how to manipulate the container will be stored directly in the system software and can be retrieved using the identification code obtained from a container's the EID tag.

The EID tag of a container also stores lot specific information, such as values that correlate to an assay component that are determined during manufacturing quality control process and specifically assigned on a lot-to-lot basis. This is generally referred to as value assignment. The EID tag also stores other pieces of information related to usage tracking (e.g., activation timestamps, status, how much material is available still, and the like), authentication parameters or keys, and data that corresponds to consumable lifecycle traceability (e.g., serial number, lot number, plant, expiration date, and the like). Thus, in sum, the EID tag stores unique lot to lot value assignments that are needed to run one or more assays using the container.

Some manufacturing data may be stored on the EID tag for the purpose of lifecycle traceability. And, to confirm container integrity, an authentication key may be stored on each EID tag, which can be compared to a predefined key stored in the control system to authenticate a container. In some implementations, particularly where additional storage is available, information such as sequences, recipes, and vial locations within a container may be stored on an EID.

Examples of types of information stored on the EID tag are as follows; however, other types of information not listed below may be stored on the EID tags. This information may include, but is not limited to, a state of the container - for example, whether the container is full, empty, partially full, and/or expired. The state of the container may also include "new", which is equivalent to full. There may also be a corresponding estimation of the number of tests that remain for the container that can be displayed on a UI accessible by the user. As the container contents are exhausted, this information is updated on the EID tag, and the updated information is displayed on the UI. If a partially full container is removed from one instrument and placed on another instrument, up-to-date information is stored on the container's EID tag so the container can be used seamlessly on the second instrument. Other types of the information stored on the EID tag may include, but are not limited to, calibration data for the container, e.g., values assigned to a manufacturing lot of material produced during a quality control (QC) process such as target values, units, and acceptance ranges that are assay type dependent; identification data for the container - for example, what the container contains or what assay the container is for; lifecycle data for the container - for example, whether the container is unused, expired, or the number of tests that can be performed given the amount of substance remaining in the container; assay data for medical diagnostic tests - for example, instructions for performing an assay using the container; container authenticity data or key(s); and/or error detection data - for example, data indicating that the system was interrupted during mixing / preparation

In some implementations, the information stored on an EID tag may also include container serial number, container lot number, container sequence number, plant identifier - for example, what facility made the container, container type identifier, container expiration date, container activation timestamp, and onboard stability (OBS) of the container - for example, the time since the container was initially pierced. The container serial number, container lot number, container sequence number, and plant identifier may be used to verify the authenticity of the container. The container EID tags may contain assay information that is readable from the container. This information may include, but is not limited to, target values and/or ranges of acceptable values for quality control (QC) and calibrator data for a given assay.

Initial values for at least some of the preceding information may be programmed or written to a container's EID tag before the container is used, e.g., at manufacturing or preparation and/or changed during use of the container. For example, as the container contents are used, the EID tag may be updated to change at least some of this information. For example, the EID tag may be updated, e.g., through writing to the tag, to change the number of tests that may be performed using the container before its expiration date. That is, as content from the container is used, fewer tests may be performed using the container. Similarly, the EID tag may be updated to change information indicating the amount of substance contained in each container after substance is removed from, or added to, the container. The current location of the container is stored in system memory as noted; however, in some implementations the container's EID tag may be updated to store the current location of the container.

The usage of dynamic tracking information on the EID tag allows a container to be removed from an instrument before it is completely used-up. As a result, separate instrument(s) may read the EID tag and use the container in the same manner as the instrument from which the container was removed. Also during normal usage, a customer may remove a container from an instrument before it has been completely used-up and then re-inserted into the same instrument at a future time (for instance, to allow time for different types of tests). A benefit of dynamic tracking is allowing the customer to view on a UI information about the container whether it is in a single instrument its entire life, re-inserted, or moved from instrument to instrument.

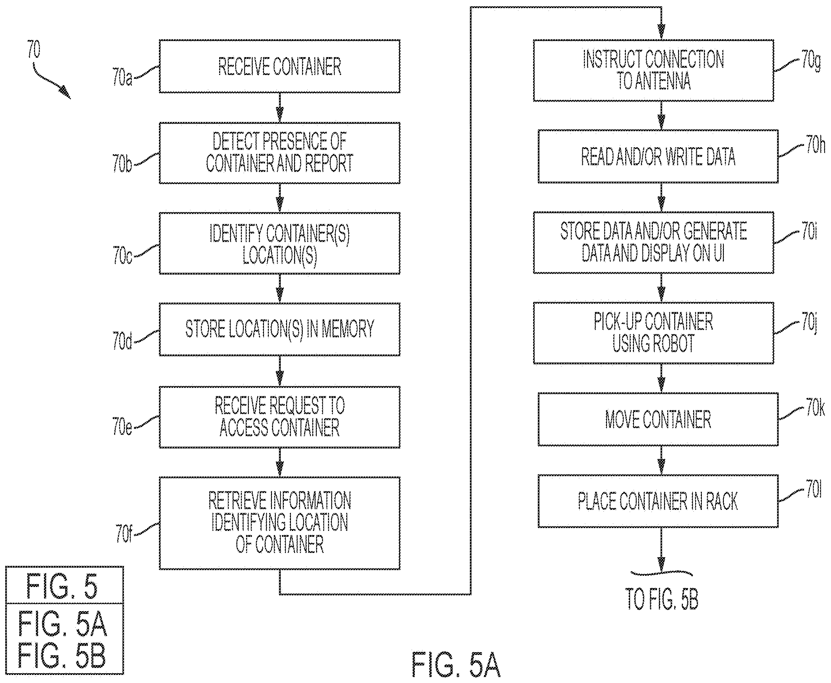

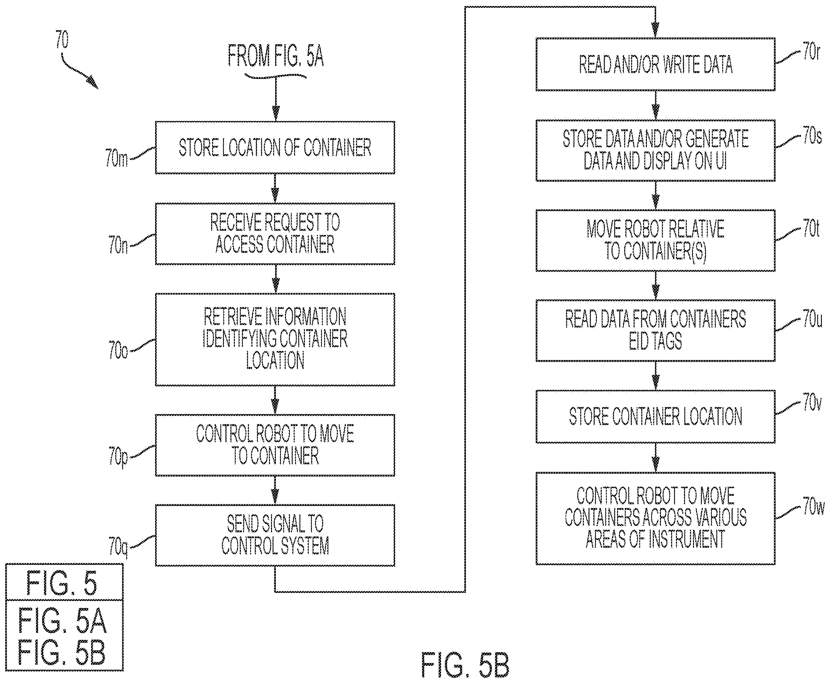

Fig. 5 is a flowchart showing an example process 70 that may be performed by or on system 10. According to process 70, the instrument receives (70a) one or more containers in area 12. The containers may be input manually or robotically, and may be input as a set or individually. For example, the containers may be loaded manually into the I/O drawer 29 of Fig. 3. The drawer is then inserted into the system. In this example, the I/O drawer includes the static RF antennas and EID circuit 36 described with respect to Fig. 3. Note that in some implementations, the system may include multiple I/O drawers, each to receive a separate set of containers.

Presence detector 64 detects (70b) the presence of at least one container in area and reports this information to the control system. The control system may then identify (70c) the locations of containers within area 12. This may be done automatically in response to detecting the presence of a container in the system, periodically, or in response to user or programmatic input to the control system. For example, at initialization, the control system may attempt to identify each container that is loaded into the system. As part of the identification process, the control system may control multiplexer 34 (Fig. 3) to select each RF antenna in succession and to attempt to read or write data using the selected RF antenna. Where reading or writing is successful - for example, data is read from or written to the EID tag without disruption or error - the control system determines that a container is present.

The control system knows the locations of the static antennas that performed the reading or writing and, from this, the control system can identify the location of the container or containers in area 12. As part of the reading operation, the control system may read an identification code stored on each container's EID. The identification code is a code that is unique to the container. The control system stores (70d) in its memory the location of containers in area 12, along with their identification codes. For example, if a container or containers is/are added, the location of those container(s) is/are stored. Also, the locations of containers known to be in area 12 may be updated if their locations have changed, e.g., moved away from the area 12, or their expected locations may be verified, e.g., using the antenna that is accessible at the changed locations. Each location within area 12 may be defined by an area identifier and coordinates within that area or general system coordinates.

The control system receives (70e) a request to access a container in area 12. For example, the control system may receive a user request from a user interface or an automated request from another computer program operating on the system. The request may be a request for information about the container or to write information to the container's EID tag. The request may identify the container by its identification code. In some implementations, the request may be for a particular type of container containing a particular type of substance. The control system may use stored information to identify a container (e.g., by identification code) that meet the criteria specified in such a quest. The control system retrieves (70f) information identifying the location of the container that is the subject of the request using the identification code.

The control system instructs the EID module 33 to connect (70g) the multiplexer 34 to the RF antenna that is within communication range of container that is the subject of the request. In a case where data is to be read (70h), this is done by selecting the static RF antenna that is capable of communicating with that container's EID tag based on stored information associating the antennas and containers described above and instructing the RF antenna to read from a part of memory on the EID tag containing the data. In this example, only the selected antenna attempts reading. The rest of the antennas are de-energized. Software running on the control system controls which antenna to be energized and used for reading. EID module 33 outputs an electrical signal to the selected RF antenna that excites the selected RF antenna to produce an RF signal in order to read data from the EID tag of the container. The read data is communicated by the selected RF antenna to the EID module, which then sends the read data to the control system. The data read from the container may be or include any of the EID tag data described herein.

In a case where data is to be written (70h), the antenna is selected in the same manner as described above and the RF signal is used to write data to the EID tag attached to container of interest. Specifically, the control system 21 provides, to the EID module, data to be written to the EID tag along with a location on the EID memory of where that data is to be written. In some examples, the data to be written is based on data from controller modules, computers, and/or user(s). The EID module 33 excites the selected RF antenna with an RF signal in order to write the data to the EID tag of the container. The data is thereby stored the memory on the EID tag. The data written to the container may be or include updates to the EID gag data described herein or, where supported, new data that is not currently on the EID tag.

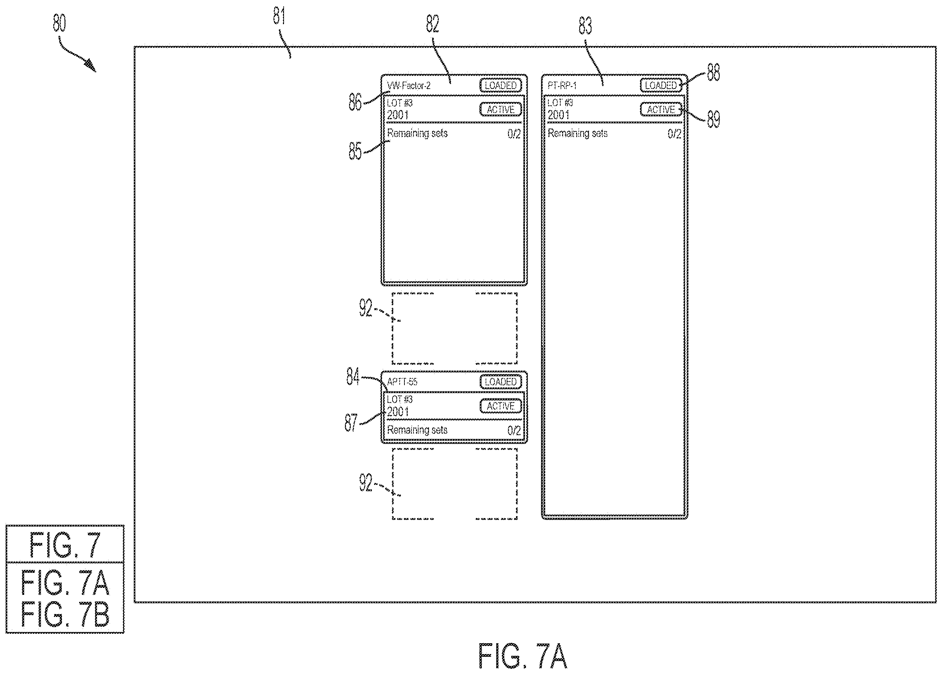

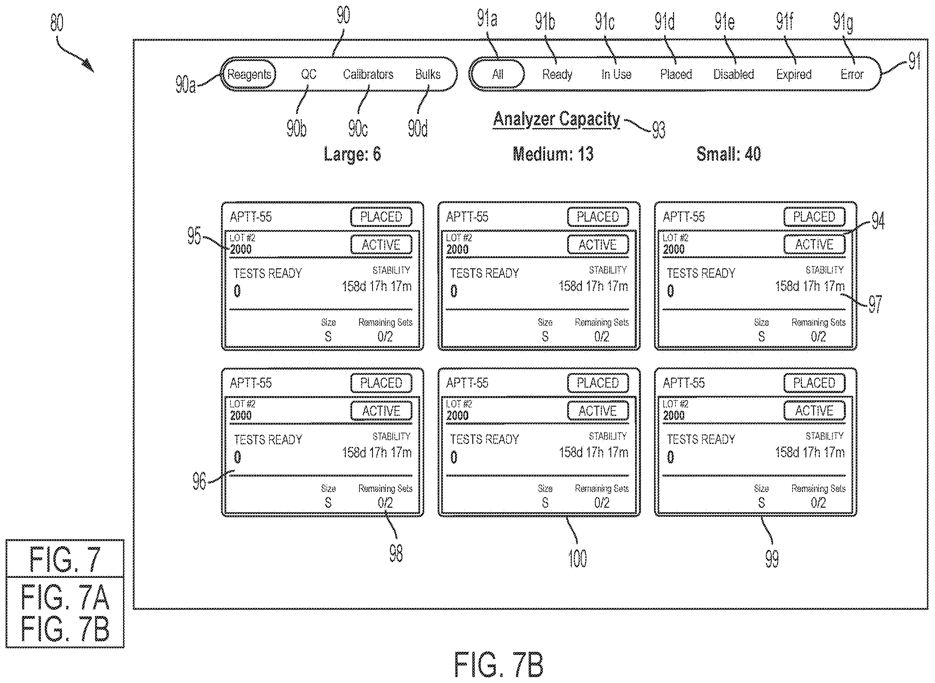

The control system may store read data and/or generate (70i) data for rendering and/or updating a user interface (UI) on a computer display that may be based at least in part information read from the EID tags or written to the EID tag. For example, the UI may present any information available from the EID tag or identify information written to the EID tag. The UI may be interactive in that a container may be selectable on a computer display. In response, information about the container may be displayed. An example of a graphical UI (GUI) is described with respect to Fig. 7 below. Other types of UIs, such as character or command interfaces may also be used as the UI.

Referring also to Fig. 1, robot 45 may be controlled by the control system to pick-up (70j) a container in area 12 and to move (70k) the container from area 12. For example, the control system may receive a user request from a user interface or an automated request from another computer program operating on the system. The request may be a request to move a container containing the desired substance from area 12 to another area, such as area 13, 14, or 15. The request may identify the container by its identification code or by assay or substance. A process for locating/identifying the container within the area 12 can be the same or similar to those described above. If the desired container does not exist, a message may be sent to the UI to alert the user. The location to which the container is to be moved may be defined by an area identifier and by a slot identifier stored in the system software, for example.

Referring also to Figs. 8 and 9, picking-up a container may include a robot, such as robot 110 moving to the location of the container and over a top of the container. The container's current location is known and associated with its identification code; accordingly, the control system instructs the robot to move using this information. At the location, assembly 150 moves downward along the Z-axis toward the container while gripper 111 is controlled to open by moving its fingers in the directions of arrows 119. As assembly 150 continues to move downward, plate 126 engages the top of the container and spring 136 compresses. This causes trigger 143 to move upwards and to block the light beam in the optical element 141. The optical switch 140 sends a signal to the control system, which instructs the robot to close gripper 111 by moving its fingers in the directions of arrows 117. The gripper closes, resulting in alignment of its juts to the notches on the container. The control system then controls assembly 150, with the container held by the grippers, to move upwards (arrow 131) along the Z-axis for movement of the container to the location to which the container is to be moved.

As noted, the control system may store data indicating a height at which each container is to engage plate 126. The data may represent, for example, the amount of Z-axis movement of assembly 150 that is needed to cause plate 126 to engage the top of the container and therefore cause trigger 143 to block the light beam in optical element 141. This data may be specific to the container and may be stored in the control system prior to operation of the instrument. The amount of Z-axis movement of assembly 150 that actually causes plate 126 to engage the top of the container and therefore causes trigger 143 to block the light beam in optical element 141 is determined by motor / encoder 152. If the trigger blocks the light beam prematurely based on a comparison of expected Z-axis movement to actual Z-axis movement, this may be an indication that the container is seated in the system incorrectly. In this case, the control system may identify an error. The robot may then be instructed to implement one or more additional attempts to pick-up the container. If these additional attempts result in an error, the control system may instruct manual or automated correction of the container position.

Similarly, if the if the trigger blocks the light beam after it is expected to, or does not block the light beam at all, based on a comparison of expected Z-axis movement to actual Z-axis movement, this may be an indication that the container is seated in the system incorrectly or that there is no container at the expected location. As was the case above, the robot may then be instructed to implement one or more additional attempts to pick-up the container. If these additional attempts result in an error, the control system may instruct manual or automated correction, e.g., through the UI.

In an example, the robot may move the container from the I/O drawer to another area of the system - for example, to area 15 of Fig. 1. During movement, force applied by plate 126 to the top of container restricts movement of the container relative to the robot, thereby reducing internal movement of the container's contents.

At area 15, robot 110 is controlled by the control system to place (70l - Fig. 5A) the container in a rack such as rack 59 shown in Fig. 4. The container may be placed in a predefined orientation so that its EID tag will always face an RFID antenna on a mobile robot - for example, in the orientation shown in Fig. 4. In implementations where the robot has multiple RF antennas or the container has multiple EIDs as described above, the container need not be placed in a specific orientation. Referring also to Figs. 8 and 9, placement of the container includes the control system instructing motor /encoder 152 to move assembly 150 downwards along Z-axis 130 (arrow 132) for a predefined distance. For example, the control system may be programmed with data identifying how far the assembly has to travel to place the container in an area. For example, the control system may be detect the placement automatically in real time, for example, when the container stops its downward travel along the Z-axis. Locations of containers or open slots within the instrument may be determined by sweeping EID tags as described herein. When assembly 150 reaches the end of its travel, the control system controls gripper 111 to move its fingers in the directions of arrows 119 to release the container. Force applied by plate 126 to the top of container may act to keep the container in place during release by the gripper. Robot 110 may then move upwards along Z-axis 130 (arrow 131) away from the container.

The control system may store (70m) the new location of the container in control system memory. For example, an area identifier and a slot identifier may be stored in association with the container's identification code in system software or memory. Any prior location(s) stored for the container may be retained in system software or memory to provide a historical record of the route that the container has traveled in the system or those prior location(s) may be deleted and only the current / most up-to-date location of the container may be retained.

The control system receives (70n) a request to access the container that was moved. For example, the control system may receive a user request from a user interface or an automated request from another computer program operating on the system. The request may be a request for information about the container or to write information to the container and may identify the container by its identification code.

The control system retrieves (70o) information identifying the location of the container that is the subject of the request. This information is associated in memory with the identification code and is retrieved using the identification code.

The control system instructs robot 45 to move to the current location of the container. In the example of Fig. 4 assuming the subject container is container 56, the control system controls robot 45 to move (70p) relative to the container array so that RF antenna on the robot is within HF NFC communication range of EID tag 44 of the container. When in communication range, the mobile robot's RF antenna sends (70q) a signal to the control system, such as the controller PCB 21. If data is to be written (70r) to the container's EID tag, the controller system sends that data to RF antenna 41 over a wired or wireless connection and the RF antenna writes the data to the EID tag as described above. If data is to be read (70r) from the EID tag, the RF antenna reads the data from the EID tag as described above and sends the data to the control system over the wired or wireless connection. Read data is then stored (70s) in software or memory and/or may be used to generate and/or update a UI.

In some implementations, the location of a container in area 40 (Fig. 4) needs to be identified or verified. Identification may be implemented by moving (70t) robot 45 and its associated RF antenna relative to one or more arrays of containers and reading (70u) data from the containers until the identification code of the container of interest is read. The current location of the container may be stored (70v) and/or an indication that a check was made may be stored in control system memory to identify the time of its last known location. In some implementations, each time there is a request or need to update container contents, the location of the container may be verified to confirm that its position has not changed. This may be implemented by moving robot 45 and its associated RF antenna to the expected position of the container and reading its EID tag. In some implementations, robot 45 and its associated RF antenna is controllable to sweep arrays of containers periodically or in response to user input in order to identify/verify container locations.

In some implementations, the system may take inventory of containers in areas by moving the mobile robot into contact with the container and confirming the proper searing of the container within the system.

Control system may control (70w) the robot to move containers into, and across, the various areas 12 to 15 in any order and at any time instructed. The operations performed following container movement to each area are as described above, depending on whether the area contains an array of static RF antennas and/or use mobile RF antennas. The RF antennas and EIDs may be used to provide information identifying the location of the containers with in the system, which the control system may store memory. Thus, tracking within the system may be implemented based on antenna location. As noted, if an antenna is at a certain location in the system and that antenna detects a code associated with a particular container, the system then knows the location of that container and stores that location in memory. The antenna acts like a positioning system and the EID tag is used to detect the presence of the container.

Within each area, data on a container's EID tag may be monitored and/or updated - for example, to reflect the current amount of substance in the container, the number of tests or volume remaining, and so forth. Accordingly, EID tag data may be used to track and/or to monitor the quantity and quality of a container. Writing information to the EID tag in particular enables tracking and/or monitoring of the container throughout the lifecycle of the container/test. The tracking and/or monitoring may occur continuously throughout use of the container in the system or it may occur intermittently or periodically in response to a user or machine command. Use of electronic mechanisms to track and/or monitor containers may reduce the need for paper-based insert sheets that have heretofore been distributed with medical diagnostic instruments. The systems and methods described herein can allow continued and automated management of the containers, assays, and tests throughout multiple rounds of testing and/or lifecycles of the containers, substances, or assays.

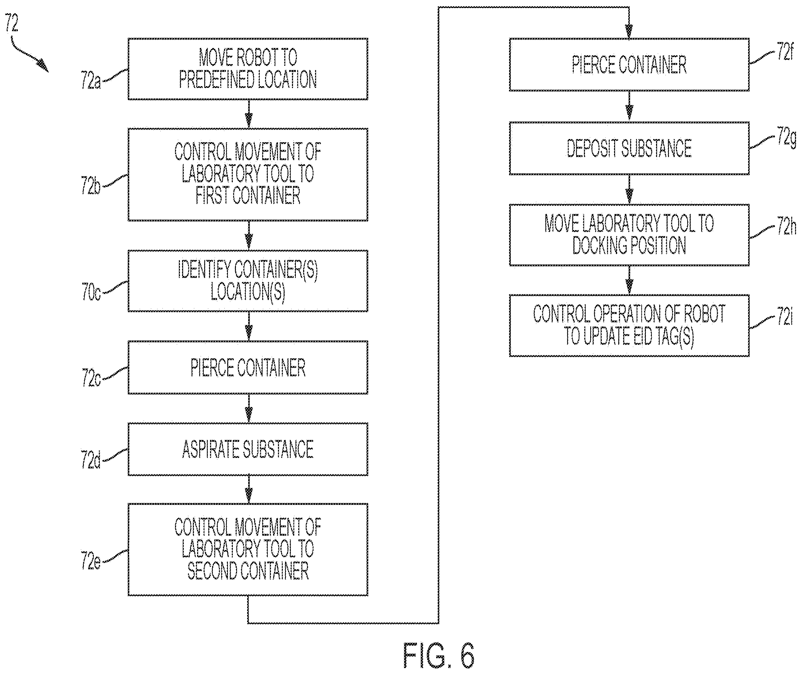

Referring to Fig. 6, in another example process 72, the control system controls a robotic laboratory tool to mix substances from different containers. The movement of the laboratory tool and the robot within the system can be coordinated, e.g., automatically by the control system. The coordination can be done based on locations of the tool and the robot, the usage needs of the tool and the robot, and/or other factors, for example, in areas 13, 14, or 15. The robot and the robotic laboratory tool may be controlled together to move out of the way of the other during their respective operations. For example, the control system may instruct the robot to move out of the way of the laboratory tool; the control system may instruct the laboratory tool to perform its operation(s) while the robot is out of the way of the laboratory tool; and then, following the operation(s) of the laboratory tool, the control system may instruct the laboratory tool to move out of the way of the robot and instruct the robot to continue its operations. In another example, the control system may instruct the laboratory tool to move out of the way of the robot; the control system may instruct the robot to perform its operation(s) while the laboratory tool is out of the way of the robot; and then, following the operation(s) of the robot, the control system may instruct the robot to move out of the way of the laboratory tool and instruct the laboratory tool to continue its operations.

In a more detailed example of such a process, prior to moving the robotic laboratory tool, the control system controls (72a) the robot 45/110 to move to a predefined location inside of or outside of the area that is out-of-the-way of the laboratory tool. That is, the robot is moved to a location that will prevent it from interfering with the operation of the laboratory tool. The location may be an X,Y axis location within the robot's area or the location may be at a Z-axis location above the laboratory tool. For example, part of the robot, such as assembly 150 of Fig. 9, may be retracted upwards in the Z dimension so that the laboratory tool (or tools) can move underneath the robot. The retraction may be such that the laboratory tool (or tools) can move underneath the robot even when the robot is holding a container.

Following movement of the robot, the control system may control (72b) the laboratory tool to move to a first container. The location of the first container may be known using the techniques described herein. The control system controls the laboratory tool to pierce the first container (72c), to aspirate (72d) substance from the first container, and to move (72e) to a second container into which that substance is to be deposited. The location of the second container may also be known using the techniques described herein. The control system controls the laboratory tool to pierce the second container (72f) and to deposit (72g) the substance in the other container.

Following operation of the laboratory tool, the control system may move (72h) the laboratory tool to a docking position that is out-of-the-way of the robot. The control system may then control operation of the robot (72i) to update the EID tag of each container using the RF antennas and the techniques described herein. For example, the EID of the second container may be updated to indicate that, e.g., the first container is ready for use by an assay. The EID of the first container may be updated to indicate, e.g., how much of the substance that was removed is left that container. The EID tag of each container may be updated to indicate when each container was first pierced, since that information bears on the expiration and onboard stability of the container.

The operations for moving the robot out-of-the-way of the laboratory tool are described with respect to laboratory tools used to move substances between containers. The operations, however, are not limited to this context and may be used with any type of laboratory tool. For example, the operations may be used with a laboratory tool that does not move any substances but rather performs other operations with respect the containers, such as controlling pressure within containers or mixing.

In some cases, the control system may cause the EID tag of the first container to store information about an error associated with the container. For example, during mixing of substances from the first and second container, the system may experience a power outage. In that case, since the system has not tracked where it is in the mixing process, there is an unrecoverable error. As a result, the EID tag of the first container is updated to indicate that the container is defective. Each time its EID tag is read, the control system knows that this container is defective and that its contents cannot be used for an assay. In some implementations, the control system may provide the error information to a user through the UI so that a user can act on the error, e.g., by removing the container from the instrument.

In some cases, containers used in one system can be moved to, and used in, a different system. For example, a container may be moved out of a first system, moved to storage, and then moved into a second system for use in that that second system. The control system may track the location and other EID stored information (e.g., substance, amount, etc. as described previously) of a container using RFID tags.

In an example, the control system may determine when the assays for which that container is designated have been completed by the first system (e.g., a first instrument). In that case, the control system may update the EID tag on the container to indicate that it is no longer needed in the first system. In this case, the number of test remaining in the container may be updated on the container EID tag in addition to any applicable expiration dates. The control system may then alert the user via the UI that the container is no longer needed on the first system. The container may then be designated for use in a second system (e.g., a second instrument). This information may be displayed on the UI - that is, the UI may indicate that the container has fulfilled its purpose in the system and can be moved to another system for use.

A user may then remove the container from the first system and place the container on an I/O drawer of the second system. That drawer may be inserted into the I/O slot of the second system and used for medical diagnostic testing in the manner described herein. The second system may read from and write to the EID tags on the container in the manner described herein in order to track the container in the other system and determine relevant information for use in the second system such as the number of tests remaining for the container, the expiration date of the container, authenticity data for the container, a state of the container, calibration data for the container, lifecycle data for the container, assay data for the medical diagnostic test, container authenticity data, and error detection data for the container, for example. Information thus may be carried from system-to-system on the EID tags.

When the expiration date has been reached, or a number of tests performed using a container has been reached, the control system may prevent use of the container. For example, the control system may provide an error message on the UI each time that container's use is attempted and/or move the container to the I/O drawer for removal by the user while sending the user a message via the UI.

In some implementations, the UI that facilitates user participation and knowledge of the automated test management described herein. Examples of information that may be included on the UI include, but are not limited to, the locations of containers in an area of the system, container IDs (identifier), container lot IDs, an indication that a container has loaded into the system properly, an indication that the container is active in the system, the number of tests remaining in a container, the locations of empty slots not containing a container, the container capacity of the instrument, the number of each sized container in the instrument, and menus allowing a user to display information be container status and/or by container content. The display of information on the UI and the ability to select which information to display provides user with the ability to identify what containers are in an instrument and their status. Selectable menus, such as menus 90 and 91 below allow users to obtain information on subset of containers in the system for which the users may have a particular interest.

To generate a UI, information obtained from EID tags may be used to identify the containers when they are in an area 12 to 15. The EID tags on the containers may then be read using the techniques described herein. Referring to Fig. 7A, an example UI 80 includes a graphical representation of an area 81 that holds three containers. Area 81 may be any of areas 12 to 15. These containers are represented graphically by UI elements 82, 83, and 84 and are shown at their locations within the instrument in area 81. Slots 91 and 92 of area 81 do not include containers and are shown as empty. The EID tags of the containers are read as described herein and information therefrom is displayed on UI 80. That information includes the number of tests 85 remaining in a container, the container ID (identifier) 86, the container lot ID 87, an indication 88 that the container has loaded into the system properly, and an indication 89 that the container is active in the system. In general, any of the information that may be obtained from the EID tags described herein may be displayed on UI 80. UI elements 82, 83, and 84 may be users-selectable to cause the UI to display such information either on UI 80 or another part (not shown) of the UI.

Referring to Fig. 7B, this part of the example UI 80 includes selectable menus 90 and 91 that allow a user to display information about containers in the system. In this example, menu 90 allows a user to select containers containing a particular substance, such as reagents 90a (e.g., ReadiPlasTin, SynthASil, Q.F.A. Thrombin, D-Dimer HS 500, or Thrombin Time), QCs - quality controls - 90b (e.g., Normal Control, Low Abnormal Control, or High Abnormal Control), calibrators 90c (e.g., Calibration Plasma, Heparin Calibrators, or HIT Calibrator), and bulks 90d (e.g., deionized (DI) water or Factor Diluent). Menu 91 allows user to select containers based on the status of the containers, such as all 91a containers in the system, containers that are ready 91b for use, containers that are currently in use 91c, containers that are placed 91d in the system but not in use, containers that are disabled 91e, containers that are expired 91f, and containers that have errors 91g associated therewith. UI 80 also identifies the capacity 93 of the system, including the number of large (L) containers in the system, the number of medium (M) containers in the system, and the number of small (S) containers in the system. In some examples, more or fewer than three different sizes of container may be accommodated by the system.

In general, any of the information that may be obtained from the EID tags described herein may be displayed on UI 80. In this example, the following information is displayed: status 94, lot number 95, number of tests 96 that the container is ready to perform, the time 97 remaining that the container's contents will remain stable, the size 99 of the container, and an element 98, such as 0/2. In this particular example, 0/2 is an example of how many "sets" are remaining. Basically, within each container, its contents are divided into sets. Each set within a container can stand-alone and has all the needed materials to perform assays. Each set is prepared independently and on an as-needed basis. So if a container has 2 sets, the first will be prepared and its on-board stability (shorter) shelf life will start. The other set will remain unprepared and at shelf life stability (longer). In the 0/2 example, zero of two sets are remaining in the container. In some implementations, individual graphical elements, such as graphical element 100, may be selected to obtain more information about given container represented by that graphical element, such as any of the information stored on the container's EID tag. The arrangement of the UI elements on the UI are examples only. There can be other elements and other arrangements

The techniques described herein is not limited to use in a medical diagnostic context, but rather may be used in any appropriate system context that involves tracking containers inside a system and between systems.

The control system described herein may be implemented using computing systems or any other appropriate computing device. The control system can be implemented, at least in part, using one or more computer program products, e.g., one or more computer program tangibly embodied in one or more information carriers, such as one or more non-transitory machine-readable media, for execution by, or to control the operation of, one or more data processing apparatus, e.g., a programmable processor, a computer, multiple computers, and/or programmable logic components.

A computer program can be written in any form of programming language, including compiled or interpreted languages, and it can be deployed in any form, including as a stand-alone program or as a module, component, subroutine, or other unit suitable for use in a computing environment. A computer program can be deployed to be executed on one computer or on multiple computers at one site or distributed across multiple sites and interconnected by a network.

Actions associated with implementing all or part of the control system can be performed by one or more programmable processors executing one or more computer programs to perform the functions described herein. All or part of the control system can be implemented using special purpose logic circuitry, e.g., an FPGA (field programmable gate array) and/or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only storage area or a random-access storage area or both. Elements of a computer (including a server) include one or more processors for executing instructions and one or more storage area devices for storing instructions and data. Generally, a computer will also include, or be operatively coupled to receive data from, or transfer data to, or both, one or more machine-readable storage media, such as mass storage devices for storing data, e.g., magnetic, magneto-optical disks, or optical disks. Machine-readable storage media suitable for embodying computer program instructions and data include all forms of non-volatile storage area, including by way of example, semiconductor storage area devices, e.g., EPROM, EEPROM, and flash storage area devices; magnetic disks, e.g., internal hard disks or removable disks; magneto-optical disks; and CD-ROM and DVD-ROM disks.

Elements of different implementations described herein may be combined to form other embodiments not specifically set forth above. Elements may be left out of the structures described herein without adversely affecting their operation. Furthermore, various separate elements may be combined into one or more individual elements to perform the functions described herein.

Further aspects, features and embodiments of the present invention are described in the following items:
1. A medical diagnostic system (10) comprising:
   one or more containers (17, 22) that are usable in a medical diagnostic test, each container (17, 22) having an identification, ID, tag (20) associated therewith, an ID tag (20) of a container (22) comprising memory (24) that is readable and writeable, the memory (24) for storing information relating to the container (22);
   antennas (32) that are configured to communicate wirelessly with ID tags (20) associated with the one or more containers (17, 22); and
   a control system (21) to store a location of the container (17) based on an identify of the antenna (32), to select the antenna (32) for communication with the ID tag (20), and to use the antenna (32) to read at least some of the information from, or to write at least some of the information to, the memory (24) on the ID tag (20).
2. The medical diagnostic system (10) of item 1, wherein an antenna among the antennas (32) is within communication range of the ID tag (20) of the container (17, 22), wherein the control system (21) in particular comprises:
   a multiplexer (34) that is controllable to select the antenna (32); and
   a read/write module (33) to implement reading at least some of the information from, or writing at least some of the information to, the memory (24) on the ID tag (20).
3. The medical diagnostic system (10) according to any one of the preceding items, wherein one or more containers (17, 22) are arranged in an array (39); and
   wherein the antennas (32) are arranged in a static array that is along the array (39) of containers such that each container (17, 22) is in physical correspondence with an antenna (32) in the static array and such that an ID tag (20) of each container (22) is in communication range of an antenna (32) in the static array.
4. The medical diagnostic system (10) according to any one of the preceding items,
   - wherein the information relating to the container (17, 22) comprises one or more of: a state of the container, calibration data for the container, identification data for the container, container manufacturer location, container expiration date, material consumed in the container, onboard stability information, assay data for the medical diagnostic test, container authenticity data, or error detection data; and/or
   - wherein the medical diagnostic system (10) is a first medical diagnostic system; and wherein the information in the memory (24) on the ID tag (20) is usable by a second medical diagnostic system that is separate from the first medical diagnostic system to incorporate the container (17, 22) into a medical diagnostic test performed by the second medical diagnostic system.
5. The medical diagnostic system (10) according to any one of the preceding items, further comprising a mobile robot (45) that is configured to move relative to the one or more containers (17, 22);
   wherein an antenna is mounted to the mobile robot (45) to cause the antenna to move relative to the one or more containers (17, 22) and, based on the movement, to communicate wirelessly with the ID tag (20); and
   wherein the control system (21) is configured to control movement of the mobile robot (45).
6. A mobile robot (45) comprising:
   a gripper (111) for grasping a container (17, 22) in a medical diagnostic system (10); and
   a mount (112) for holding an antenna (114) configured to communicate wirelessly with electronic identification, EID, tags on containers in the medical diagnostic system (10);
   wherein the mobile robot (45) is configured to move the container between different areas (12, 13, 14, 15) of the medical diagnostic system (10).
7. The mobile robot (45) of item 6, wherein the mobile robot (45) comprises an element (126) to apply force to a part of the container (120) during movement in order to stabilize the container during the movement, wherein the element (126) in particular comprises a spring-loaded plate.
8. The mobile robot (45) of item 6 or item 7, wherein the mobile robot (45) is configured to move in three dimensions; and
   wherein the mobile robot (45) comprises a switch (124) that detects engagement with the container, wherein the switch (124) is in particular configured to output a signal in response to engagement between the container and the mobile robot (45).
9. A system comprising:
   the mobile robot (45) according to any one of items 6 to 8; and
   a control system (21) to control operation of the mobile robot (45), the control system (21) being configured to detect the signal output by switch (124).
10. The system of item 9, wherein the control system (21) is configured to track movement of the mobile robot (45) relative to the container, to detect that the signal was not output when expected, and to output an error signal in response to detecting that the signal was not output when expected.
11. The system of item 9 or item 10, further comprising:
   a robotic laboratory tool (62) configured to move between containers to transfer a substance from a first container to a second container, wherein the control system (21) is in particular configured to control movement of the mobile robot (45) so that the mobile robot (45) is out of the way of the robotic laboratory tool (62) during operation of the robotic laboratory tool (62).
12. A user interface, UI, for a medical diagnostic system (10), the UI comprising:
   a menu for selecting one or more containers (17, 22) in the medical diagnostic system (10) based on a substance in the containers or a status of the containers; and
   a display containing information about the one or more containers (17, 22) selected.
13. The UI of item 12, wherein the one or more containers (17, 22) are represented graphically; and
   wherein the information comprises at least one of the following: locations of the one or more containers (17, 22) in an area (12, 13, 14, 15) of the medical diagnostic system (10), container identifiers, container lot identifiers, an indication that a container has loaded into the medical diagnostic system, an indication that a container is active in the medical diagnostic system (10), a number of tests remaining in a container in the medical diagnostic system (10), locations of empty slots not containing a container in the medical diagnostic system (10), container capacity of the medical diagnostic system (10), or a number of each sized container in the medical diagnostic system (10).
14. A method for use in a medical diagnostic system (10), comprising:
   selecting a first antenna among multiple antennas (32), the first antenna being associated with an identification, ID, tag (20) of a container (22) that is usable in a medical diagnostic test, the ID tag (20) comprising memory (24) that is readable and writeable, the memory (24) for storing information relating to the container (22);
   performing at least one of the following operations: reading at least some of the information from the memory (24) using the first antenna, or writing at least some of the information to the memory (24) using the first antenna;
   moving the container (22) from a first location of the medical diagnostic system (10) to a second location of the medical diagnostic system (10) using a mobile robot (45); and
   communicating with the ID tag (20) of the container (22) using a second antenna mounted to the mobile robot (45) at least to update one or more of: the memory (24) on the ID tag (20) or a control system (21) to provide information from the ID tag (20).
15. The method of item 14, further comprising:
   storing, in memory, a first location of the container (22) based on an identity of the first antenna; and
   storing, in memory, the second location of the container (22).

## Claims

1. A medical diagnostic system (10) comprising:
one or more containers (17, 22) that are usable in a medical diagnostic test, each container (17, 22) having an identification, ID, tag (20) associated therewith, an ID tag (20) of a container (22) comprising memory (24) that is readable and writeable, the memory (24) for storing information relating to the container (22);
a mobile antenna (41; 114) configured to communicate wirelessly with ID tags (20) associated with the one or more containers (17, 22);
a mobile robot (45) that is configured to move relative to the one or more containers (17, 22);
wherein the mobile antenna (41; 114) is mounted to the mobile robot (45) to cause the mobile antenna (41; 114) to move relative to the one or more containers (17, 22) and, based on the movement, to communicate wirelessly with the ID tag (20); and
a control system (21) to store a location of the container (17), to use the mobile antenna (41; 114) to read at least some of the information from, or to write at least some of the information to, the memory (24) on the ID tag (20), and to control movement of the mobile robot (45).

2. The medical diagnostic system (10) according to claim 1,
- wherein the information relating to the container (17, 22) comprises one or more of: a state of the container, calibration data for the container, identification data for the container, container manufacturer location, container expiration date, material consumed in the container, onboard stability information, assay data for the medical diagnostic test, container authenticity data, or error detection data; and/or
- wherein the medical diagnostic system (10) is a first medical diagnostic system; and wherein the information in the memory (24) on the ID tag (20) is usable by a second medical diagnostic system that is separate from the first medical diagnostic system to incorporate the container (17, 22) into a medical diagnostic test performed by the second medical diagnostic system.

3. The medical diagnostic system (10) according to any one of the preceding claims, wherein the mobile robot (45) is mounted to tracks (46 and 47) to move along tracks (46 and 47).

4. The medical diagnostic system (10) according to any one of the preceding claims, wherein the mobile robot (45) comprises:
a gripper (111) for grasping a container (17, 22) in the medical diagnostic system (10); and
a mount (112) for holding the antenna (41; 114) configured to communicate wirelessly with electronic identification, EID, tags on containers in the medical diagnostic system (10);
wherein the mobile robot (45) is configured to move the container between different areas (12, 13, 14, 15) of the medical diagnostic system (10).

5. The medical diagnostic system (10) of claim 4, wherein the mobile robot (45) comprises an element (126) to apply force to a part of the container (120) during movement in order to stabilize the container during the movement, wherein the element (126) in particular comprises a spring-loaded plate.

6. The medical diagnostic system (10) of claim 4 or claim 5, wherein the mobile robot (45) is configured to move in three dimensions; and
wherein the mobile robot (45) comprises a switch (124) that detects engagement with the container, wherein the switch (124) is in particular configured to output a signal in response to engagement between the container and the mobile robot (45).

7. The medical diagnostic system (10) of claim 6, wherein the control system (21) is further configured to detect the signal output by switch (124).

8. The medical diagnostic system (10) of claim 7, wherein the control system (21) is configured to track movement of the mobile robot (45) relative to the container, to detect that the signal was not output when expected, and to output an error signal in response to detecting that the signal was not output when expected.

9. The medical diagnostic system (10) of claim 7 or claim 8, further comprising:
a robotic laboratory tool (62) configured to move between containers to transfer a substance from a first container to a second container, wherein the control system (21) is in particular configured to control movement of the mobile robot (45) so that the mobile robot (45) is out of the way of the robotic laboratory tool (62) during operation of the robotic laboratory tool (62).

10. The medical diagnostic system (10) of any one of claims 7 to 9, wherein the control system (21) controls the mobile robot (45) to move over other arrays of containers and controls the mobile antenna (41; 114) to read and/or write data to and/or from ID tags (20).

11. The medical diagnostic system (10) of claim 10, wherein the control system (21) controls the mobile robot (45) to move at a rate of movement calibrated to enable communication with the ID tags (20) to implement the reading or writing.

12. A user interface, UI, for a medical diagnostic system (10) according to any one of the preceding claims, the UI comprising:
a menu for selecting one or more containers (17, 22) in the medical diagnostic system (10) based on a substance in the containers or a status of the containers; and
a display containing information about the one or more containers (17, 22) selected.

13. The UI of claim 12, wherein the one or more containers (17, 22) are represented graphically; and
wherein the information comprises at least one of the following: locations of the one or more containers (17, 22) in an area (12, 13, 14, 15) of the medical diagnostic system (10), container identifiers, container lot identifiers, an indication that a container has loaded into the medical diagnostic system, an indication that a container is active in the medical diagnostic system (10), a number of tests remaining in a container in the medical diagnostic system (10), locations of empty slots not containing a container in the medical diagnostic system (10), container capacity of the medical diagnostic system (10), or a number of each sized container in the medical diagnostic system (10).

14. A method for use in a medical diagnostic system (10), comprising:
moving a mobile antenna (41; 114) mounted on or associated with a mobile robot (45) relative to an identification, ID, tag (20) of a container (22) that is usable in a medical diagnostic test, the ID tag (20) comprising memory (24) that is readable and writeable, the memory (24) for storing information relating to the container (22);
performing at least one of the following operations: reading at least some of the information from the memory (24) using the mobile antenna (41; 114), or writing at least some of the information to the memory (24) using the mobile antenna (41; 114);
moving the container (22) from a first location of the medical diagnostic system (10) to a second location of the medical diagnostic system (10) using the mobile robot (45); and
communicating with the ID tag (20) of the container (22) using mobile antenna (41; 114) at least to update one or more of: the memory (24) on the ID tag (20) or a control system (21) to provide information from the ID tag (20).

15. The method of claim 14, further comprising:
storing, in memory, a first location of the container (22) based on an identity of the first antenna; and
storing, in memory, the second location of the container (22).
